Europäisches Patentamt

European Patent Office

Office européen des brevets

(11) Veröffentlichungsnummer: **0 028 316**
**B1**

(12) **EUROPÄISCHE PATENTSCHRIFT**

(45) Veröffentlichungstag der Patentschrift:
**27.12.84**

(21) Anmeldenummer: **80105968.4**

(22) Anmeldetag: **02.10.80**

(51) Int. Cl.³: **C 07 D 239/48**

(54) Verfahren zur Herstellung von 2,4-Diaminopyrimidin.

(30) Priorität: **02.11.79 DE 2944145**

(43) Veröffentlichungstag der Anmeldung:
**13.05.81 Patentblatt 81/19**

(45) Bekanntmachung des Hinweises auf die Patenterteilung:
**27.12.84 Patentblatt 84/52**

(84) Benannte Vertragsstaaten:
**BE DE FR GB IT NL**

(56) Entgegenhaltungen:
**DE - A - 1 545 966**
**US - A - 4 033 962**

**CHEMICAL ABSTRACTS, Band 82, 1975, Seite 433, Zusammenfassung 43323t, Columbus, Ohio, USA, FERRIS J.P.: "Chemical evolution. XVIII. Synthesis of pyrimidines from guanidine and cyanoacetaldehyde" J. Org. Chem. 26 (1961), S. 2770-2778**

(73) Patentinhaber: **DYNAMIT NOBEL AKTIENGESELLSCHAFT, Postfach 1209, D-5210 Troisdorf, Bez. Köln (DE)**

(72) Erfinder: **Hönigschmid-Grossich, Rüdiger, Dr., Kasseler Weg 12, D-5216 Niederkassel 3 (DE)**
Erfinder: **Schrage, Klaus, Dr., Pfarrer-Wichert-Strasse 53, D-5330 Königswinter-Uthweiler (DE)**
Erfinder: **Vogt, Wilhelm, Dr., Neuenhöfer Allee 54, D-5000 Köln-Sülz (DE)**

## Beschreibung

Die bekannten Synthesen von 2,4-Diaminopyrimidin gehen entweder von 2,4-Dihydroxypyrimidin (= Uracil) aus, in dem die Hydroxygruppen zuerst gegen Halogen- und evtl. Alkylthio- oder Alkoxygruppen ausgetauscht werden, die dann ihrerseits bei drastischen Temperaturen zwischen 180–200 °C durch Aminogruppen substituiert werden müssen (US-A-2 416 614; CA 41 [1947], 3493; T.B. Johnson u.a. Amer. Chem. J. 34 [1905], 175; Chem. Zbl. 1905 II, 1353; J.H. Clark u.a. J. Amer. Chem. Soc. 68 [1946], 96; CA 70 [1969], 68408 und CA 73 [1970], 14871), oder man geht von in 5- oder 6-Stellung substituierten 2,4-Diaminopyrimidinen aus, in denen Substituenten in 5- oder 6-Stellung, wie Halogen, Mercapto- oder Cyanogruppen, reduktiv bzw. durch Verseifung mit nachfolgender Decarboxylierung mit mässigen Ausbeuten entfernt werden müssen (E. Büttner, Ber. 36 [1903], 2227; H.N. Schlein u.a. Chem. a. Ind. [London] 1964, 418; CH-A-507 243) oder man geht von β-Chloracrylnitril aus, das seinerseits in mehreren Stufen aus Chloracetaldehyd hergestellt wird und bei der Umsetzung mit Guanidin 2,4-Diaminopyrimidin nur mit 16,4% Ausbeute liefert (W.J. Fanshawe u.a. J. Org. Chem. 30 [1965], 1278). Diese Synthesen sind durch mehrstufige Herstellwege umständlich und liefern zudem nur geringe Gesamtausbeuten. Die Synthese von 2,4-Diaminopyrimidin durch Umsetzung von β-Äthoxyacrylnitril mit Guanidin in Gegenwart von Alkalialkoholat als Kondensationskatalysator im Molverhältnis Nitril : Guanidin : Alkalialkoholat = 1 : 1 : 0,1 ist bekannt (B. Roth u.a. J. Org. Chem. 26 [1961] 2770), gedoch wird ebenfalls nur eine geringe Ausbeute von 57% erhalten.

Synthesewege zu Pyrimidinderivaten mit Substituenten in 5-Position nach DE-A-1 545 966 bzw. US-A-4 033 962 haben die Besonderheit, dass die durch Substitutionsreaktionen gefährdete 5-Position blockiert ist (vgl. D.J. Brown «The Pyrimidins», Vol. 16 Heterocyclic Compounds, Intersience Publ.).

Gegenstand der Erfindung ist ein Verfahren zur Herstellung von 2,4-Diaminopyrimidin durch Umsetzung eines durch OR in β-Stellung substituierten Acrylnitrils der Formel $NC-CH_2 = CH-OR$ oder des entsprechenden Cyanacetaldehydacetals der Formel $NC-CH_2-CH(OR)_2$ oder eines Gemisches der Acrylnitrile und Acetale mit Guanidin, worin R im Falle der Acrylnitrile und Acetale einen geradkettigen oder verzweigten Alkylrest mit 1 bis 6 C-Atomen, einen Benzyl- oder Arylrest bedeuten oder gegebenenfalls, im Falle der Acetale, beide Reste R einen gemeinsamen Alkylrest mit 3 bis 6 C-Atomen bilden, dadurch gekennzeichnet, dass die Kondensation in Abwesenheit eines Kondensationskatalysators mit einem Molverhältnis Nitril : Guanidin von 1 : 1,1 bis 1 : 1,2 durchgeführt wird.

Durch den geringen Überschuss von 10 bis 20% an Guanidin erfolgt trotz Abwesenheit eines Kondensationskatalysators (siehe dagegen Roth...)

eine ganz erhebliche Verbesserung der Ausbeute auf ca. 90 bis 95%.

Die Ausgangsstoffe sind durch Carbonylierung von Acetonitril nach der DE-A-2 753 322 und nachfolgende Alkylierung leicht zugängig.

Für Rinder Gruppe –OR werden die einfachsten und leicht beschaffbaren Substituenten, besonders die Äthyl-, gegebenenfalls auch die Methyl- oder Isoporpyl-Gruppe, entschieden bevorzugt.

Unter den Alkylresten sind solche mit 2 bis 6 C-Atomen bevorzugt. Bevorzugte Arylreste sind einkernige Arylreste, besonders Phenyl. Soweit in Acetalen beide Reste R miteinander verknüpft sind, bilden diese bevorzugt einen Alkylenrest mit 3 bis 6 C-Atomen.

Die Reaktionstemperatur kann zwischen 20 und 200 °C liegen. Bevorzugt ist der Temperaturbereich zwischen 50 und 100 °C.

Normaldruck oder Eigendruck der Komponenten ist wegen der einfachen Durchführung bevorzugt. Hohe Drücke schaden jedoch nicht.

Als Lösungsmittel kommen Alkohole, wie Äthanol, Isopropanol, tert. Butanol, Methylglykol, Äther, wie Dimethoxyäthan, tert. Amine, wie Triäthylamin, Ketone wie Aceton, weiterhin aprotische Lösungsmittel wie Acetonitril, Dimethylformamid, Dimethylsulfoxid oder Gemische dieser Lösungsmittel in Frage.

Die Kondensation kann auch in Abwesenheit von Lösungsmitteln durchgeführt werden. Zur besseren Handhabung des Reaktionsgemisches empfiehlt sich aber ein Lösungsmittel, in dem die Reaktionspartner vollständig oder teilweise gelöst sind, wobei sich die Ausgangskonzentration des Nitrils ≥ 1 Mol/kg Lösung, besonders 1,4 bis 3 Mol/kg Lösung als vorteilhaft erweist. Am Beginn der Reaktion braucht noch keine homogene Lösung bestehen, sofern nach Einsetzen der Reaktion eine homogene Lösung entsteht, in der die Reaktion rascher abläuft.

Man kann auch von verdünnteren Reaktionslösungen als oben genannt ausgehen und durch destillatives Einengen die Lösungsmittel entfernen bis Sumpftemperaturen von 70 bis 100 °C erreicht sind. Die Synthese ist in diesen Fällen in 0,5 bis 2 Stunden beendet.

Nach Entfernen des Lösungsmittels kann aus dem Rohprodukt durch Extraktion mit Äther, aliphatischen oder aromatischen Kohlenwasserstoffen nicht umgesetztes Nitril extrahiert werden, das bei einem nachfolgenden Ansatz erneut eingesetzt werden kann. Das rohe 2,4-Diaminopyrimidin, das bereits mit einer Reinheit von 85–95% anfällt, kann z.B. durch Umkristallisation aus einem azeotropen Gemisch Acetonitril/Toluol mit nachfolgender Zugabe von Toluol gereinigt werden.

2,4-Diaminopyrimidin findet u.a. Verwendung bei der Synthese des therapeutisch wichtigen 2,4-Diamino-5-(3,4,5-Trimethoxybenzyl)-pyrimidins durch Reaktion mit substituiertem Benzyldimethylamin (GB-A-1 128 234), oder durch Reaktion mit 3,4,5-Trimethoxybenzoylchlorid (CH-A-507 243).

Vergleichsbeispiel 1

0,1 Mol Guanidinhydrochlorid wurde in absolutem Äthanol bei Raumtemperatur mit 0,11 Mol 14,6 gewichtsprozentiger äthanolischer Natriumäthylatlösung versetzt, nach 10 Min. 0,1 Mol β-Äthoxyacrylnitril zugegeben. Die Äthanolmenge wurde derart bemessen, dass die Konzentration an Nitril bzw. die Summe der Konzentrationen des Nitrils und des Reaktionsproduktes 1,23 Mol/kg Lösung betrug. Molverhältnisse Nitril : Guanidin : Natriumäthylat = 1 : 1 : 0,1. Anschliessend wurde 7 Stunden am Rückfluss gekocht, dann das Natriumchlorid abfiltriert und mit absolutem Äthanol gewaschen. Im mit dem Waschalkohol vereinigten Filtrat wurden 0,052 Mol Guanidin und Natriumalkoholat und 0,054 Mol 2,4-Diaminopyrimidin gefunden. Ausbeute = 54%.

Vergleichsbeispiel 2

Das Vergleichsbeispiel wurde unter den dort angegebenen Bedingungen und Stoffmengen, jedoch mit 0,1 statt 0,11 Mol äthanolischer Natriumäthylatlösung wiederholt, d.h. ohne Überschuss an Natriumäthylat (Molverhältnis Nitril : Guanidin : Alkoholat = 1 : 1 : 0). Das Natriumäthylat wird folglich zur Freimachung des Guanidins aus dem Salz vor Beginn der Reaktion verbraucht. Es wurden 0,0605 Mol 2,4-Diaminopyrimidin gefunden. Ausbeute = 60,5%.

Vergleichsbeispiel 3

0,123 Mol Guanidin wurde nach Suspendieren in 70 ml Acetonitril mit 0,123 Mol β-Äthoxyacrylnitril versetzt, dann das Gemisch 6 Stunden zum Rückfluss erhitzt. Anschliessend wurde das Acetonitril im Vakuum abgezogen, der Rückstand mit Chloroform extrahiert. Man erhielt 8,40 g 2,4-Diaminopyrimidin mit einer Reinheit von 97,9%; Schmp. 152,2 °C. Ausbeute = 60,7%.

Durch Lösen in einem heissen Gemisch aus Acetonitril/Toluol, dessen Zusammensetzung dem Azeotrop der beiden Komponenten entspricht, Filtrieren in der Hitze und Zufügen von Toluol in der Kälte bis zum Erreichen eines Volumenverhältnisses Acetonitril : Toluol = 1 : 2,5 bis 3 fiel 2,4-Diaminopyrimidin mit einer Reinheit von 99,0% aus. Schmp. 153–153,5 °C.

Beispiel 1

(Molverhältnis Guanidin : Nitril = 1,15 : 1) innerhalb 3 Stunden wurde zu einer 65–70 °C warmen Lösung von 0,5 Mol β-Äthoxyacrylnitril (1,51 Mol/kg Lösung) in 125 g Acetonitril ein Gemisch von 37,78 g eines etwa 90gewichtsprozentigen Guanidins (0,575 Mol) in 100 g Acetonitril und 20 g Dimethylformamid zugetropft. Nach 2,5 Stunden Rühren der Reaktionsmischung bei ca. 70 °C wurde das Lösungsmittel abdestilliert bis die Sumpftemperatur ca. 100 °C erreicht. Der Rückstand wurde in 100 ml Toluol ca. 1 Stunde bei 60 °C digeriert, dabei fiel ein gelblicher Feststoff aus. Man erhielt nach Abtrennen 58,34 g rohes 2,4-Diaminopyrimidin mit einer Reinheit = 89,5%, Schmp. 145–146 °C. Ausbeute = 94,8%.

Die Umkristallisation erfolgte durch Lösen in einem heissen Gemisch aus Acetonitril/Toluol, dessen Zusammensetzung dem Azeotrop der beiden Komponenten entspricht, Abfiltrieren in der Hitze und Ausfällen durch Zufügen von Toluol in der Kälte bis zum Erreichen eines Volumenverhältnisses Acetonitril : Toluol = 1 : 2,5 bis 3. Reinheit: ca. 100%. Schmp. 152–153 °C.

Beispiel 2

Zu einer 68–70 °C warmen Lösung von 0,10 Mol β-Äthoxyacrylnitril in 25 g Isopropanol wurde während 1,5 Stunden eine Lösung von 7,23 g etwa 94gewichtsprozentigen Guanidins (0,115 Mol) in 20 g Isopropanol zugetropft. Molverhältnis β-Äthoxyacrylnitril : Guanidin = 1 : 1,15; Konzentration des Nitrils bzw. Summe der Konzentrationen des Nitrils und des Reaktionsproduktes = 1,61 Mol/kg Lösung. Anschliessend wurde 2,5 Stunden bei 69–70 °C gerührt, dann bei Normaldruck das Lösungsmittel abdestilliert bis die Sumpftemperatur 98 °C erreichte. Beim Abkühlen erstarrte der Rückstand zu einem gelb-braunen Kristallkuchen, von dem Reste an Flüchtigem bei max. 100 °C Badtemperatur im Vakuum, entfernt, nicht umgesetztes β-Äthoxyacrylnitril mit 20 g Toluol in der Wärme extrahiert wurden. Man erhielt 11,56 g 2,4-Diaminopyrimidin, Reinheit: 92,1%, Schmp. 148–150 °C, Ausbeute = 96,7%.

Beispiele 3–6

Der Ansatz des Beispiels 2 wurde wiederholt, wobei als Lösungsmittel anstelle von Isopropanol gleiche Einsatzmengen von tert. Butanol (Beispiel 3), Dimethoxyäthan (Beispiel 4), Triäthylamin (Beispiel 5) und Aceton (Beispiel 6) verwendet wurden. Dem zudosierten Guanidin wurde neben den genannten Lösungsmitteln etwas Dimethylformamid zugegeben, um eine vollständige Auflösung bzw. bessere Suspendierung (im Falle des Triäthylamins) zu erreichen.

| Konzentration des Nitrils bzw. Summe der Konzentrationen an Nitril und Reaktionsprodukt Mol/kg Lösung | Reaktionstemp. | 2,4-Diamino-pyrimidin Menge in g; Reinheit | Schmp. °C | Ausbeute % |
|---|---|---|---|---|
| 1,50 (Beispiel 3) | 70 | 11,81 g/86,6% | 144–146 | 92,9 |
| 1,45 (Beispiel 4) | 80 | 10,54 g/96,1% | 147–149 | 92,0 |
| 1,50 (Beispiel 5) | 90 | 11,38 g/89,4% | 143–145 | 92,4 |
| 1,50 (Beispiel 6) | 55 | 10,89 g/92,1% | 143–145 | 91,1 |

Beispiel 7

10,93 g ca. 94,5gewichtsprozentiges Guanidin (0,175 Mol), in 30 g Acetonitril suspendiert, wurden zu einer ca. 65°C warmen Lösung von 0,15 Mol β-Isopropoxyacrylnitril in 40 g Acetonitril langsam zudosiert. Molverhältnis β-Isopropoxyacrylnitril : Guanidin = 1 : 1,17; Konzentration an Nitril bzw. Summe der Konzentrationen an Nitril und Reaktionsprodukt = 1,54 Mol/kg Lösung. Nachfolgend wurde 1,25 Stunden bei 68–70°C gerührt, dabei färbte sich die Reaktionsmischung hellbraun und ein gelber Feststoff setzte sich ab. Zur Aufarbeitung wurde Lösungsmittel bis zum Erreichen einer Sumpftemperatur von 95°C abdestilliert, dann wurde der Destillationsrückstand in 30 g n-Heptan 1 Stunde bei ca. 60°C digeriert. Es fielen 16,70 g rohes 2,4-Diaminopyrimidin mit einer Reinheit von 85,7% an; Schmp. 139–144°C. Ausbeute = 86,6%. Durch Umkristallisation nach der im Beispiel 1 beschriebenen Weise liess sich die Reinheit auf 97,6% erhöhen. Der Heptanextrakt enthielt ca. 0,01 Mol β-Isopropoxyacrylnitril und etwas Acetonitril.

Beispiel 8

Eine Lösung von 0,089 Mol Guanidin und 0,074 Mol Cyanacetaldehydäthylacetal in 50 g absolutem Äthanol wurden 2,5 Stunden zum Rückfluss erhitzt.

Nach Abdestillieren des Äthanols bei Normaldruck, extrahieren nicht umgesetzten Acetals mit Toluol entsprechend Beispiel 3 erhält man 8,0 g 2,4-Diaminopyrimidin. Ausbeute = 87,4%, Reinheit: 90%.

**Patentansprüche**

1. Verfahren zur Herstellung von 2,4-Diaminopyrimidin durch Umsetzung eines durch OR in β-Stellung substituierten Acrylnitrils der Formel CH–CH$_2$ = CH–OR oder des entsprechenden Cyanacetaldehydacetals der Formel NC–CH$_2$–CH(OR)$_2$ oder eines Gemisches der Acrylnitrile und Acetale mit Guanidin, worin R im Falle der Acrylnitrile und Acetale einen geradkettigen oder verzweigten Alkylrest mit 1 bis 6 C-Atomen, einen Benzyl- oder Arylrest bedeuten oder gegebenenfalls, im Falle der Acetale, beide Reste R einen gemeinsamen Alkylrest mit 3 bis 6 C-Atomen bilden, dadurch gekennzeichnet, dass die Kondensation in Abwesenheit eines Kondensationskatalysators mit einem Molverhältnis Nitril : Guanidin von 1 : 1,1 bis 1 : 1,2 durchgeführt wird.

2. Verfahren nach Anspruch 1, dadurch gekennzeichnet, dass die Kondensation bei einer Temperatur zwischen Raumtemperatur und 200°C durchgeführt wird.

3. Verfahren nach einem der Ansprüche 1 oder 2, dadurch gekennzeichnet, dass die Konzentration des Nitrils bzw. der Reaktionsprodukte mindestens 1 Mol je kg Lösung beträgt.

**Claims**

1. Process for the preparation of 1,4-diaminopyrimidine by reaction of an acrylonitrile of formula NC–CH$_2$ = CH–OR substituted in the β-position, or the corresponding cyanoacetaldehydacetal of formula NC–CH$_2$–CH(OR)$_2$ or a mixture of the acrylonitrile and acetal, with guanidine, wherein R, in the case of the acrylonitrile and acetal, denotes a straight-chained or branched alkyl residue with 1–6 C-atoms, a benzyl or aryl residue, or, optionally in the case of the acetal, both residues R form a common alkyl residue with 3–6 C-atoms, characterised in that the condensation is carried out in the absence of a condensation catalyst, with a molar ratio of nitrile : guanidine of 1 : 1.1 to 1 : 1.2.

2. Process according to claim 1, characterised in that the condensation is carried out at a temperature between room temperature and 200°C.

3. Process according to one of claims 1 or 2, characterised in that the concentration of the nitrile or the reaction product amounts to at least 1 mole per kg of solution.

**Revendications**

1. Procédé de fabrication de 2,4-diaminopyrimidine par réaction d'un acrylonitrile substitué par un OR en position β, de formule NC–CH$_2$ = CH–OR, ou de l'acétal de cyanacétaldéhyde correspondant de formule NC–CH$_2$–CH(OR)$_2$, ou d'un mélange des acrylonitriles et des acétals, avec de la guanidine, R dans le cas des acrylonitriles et des acétals signifiant un radical alcoyle à chaîne droite ou ramifiée ayant 1 à 6 atomes de carbone, un radical benzyle ou aryle, ou éventuellement, dans le cas des acétals, les deux radicaux R formant un radical alcoyle commun ayant 3 à 6 atomes de carbone, caractérisé en ce qu'on effectue la condensation en l'absence d'un catalyseur de condensation avec un rapport molaire nitrile : guanidine de 1 : 1,1 à 1 : 1,2.

2. Procédé selon la revendication 1, caractérisé en ce qu'on effectue la condensation à une température entre la température ambiante et 200°C

3. Procédé selon l'une des revendications 1 ou 2, caractérisé en ce que la concentration du nitrile ou des produits de réaction s'élève au moins à 1 mole par kg de solution.